(19) Europäisches Patentamt
European Patent Office
Office européen des brevets

(11) **EP 3 040 086 B1**

(12) **EUROPÄISCHE PATENTSCHRIFT**

(45) Veröffentlichungstag und Bekanntmachung des Hinweises auf die Patenterteilung:
**03.05.2023 Patentblatt 2023/18**

(21) Anmeldenummer: **14200589.1**

(22) Anmeldetag: **30.12.2014**

(51) Internationale Patentklassifikation (IPC):
**A61L 15/18** *(2006.01)* **A61L 15/46** *(2006.01)*

(52) Gemeinsame Patentklassifikation (CPC):
**A61L 15/18; A61L 15/46;** A61L 2300/102; A61L 2300/104

(54) **Wundsystem**

Wound system

Système de soin de blessure

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**06.07.2016 Patentblatt 2016/27**

(73) Patentinhaber: **Paul Hartmann AG**
**89522 Heidenheim (DE)**

(72) Erfinder: **Smola, Hans**
**89522 Heidenheim (DE)**

(74) Vertreter: **Ter Meer Steinmeister & Partner Patentanwälte mbB**
**Nymphenburger Straße 4**
**80335 München (DE)**

(56) Entgegenhaltungen:
WO-A1-92/11043       WO-A1-2011/141454
WO-A1-2014/178945    WO-A2-2005/004982
WO-A2-2010/000450    US-B1- 7 214 847

• Jaideep Banerjee ET AL: "Improvement of Human Keratinocyte Migration by a Redox Active Bioelectric Dressing", PLoS ONE, vol. 9, no. 3, 1 March 2014 (2014-03-01), pages 1-14, XP055273094, DOI: 10.1371/journal.pone.0089239

**Beschreibung**

Die Erfindung betrifft ein Wundsystem, welches insbesondere in der Wundbehandlung während der Epithelisierungsphase eingesetzt wird. Das Wundsystem umfasst eine erste Schicht, die zwei räumlich voneinander getrennte Stoffe mit unterschiedlichen Standardpotentialen enthält, sowie eine weitere Schicht, die dazu geeignet ist, die Wunde feucht zu halten. Das beschriebene Wundsystem kann insbesondere bei der feuchten Wundbehandlung eingesetzt werden. Darüber hinaus betrifft die Erfindung ein Stoffgemisch aus einem ersten und einem zweiten Stoff, wobei der erste und der zweite Stoff räumlich voneinander getrennt sind, sowie unterschiedliche Standardpotentiale aufweisen, wobei das Stoffgemisch in Kombination mit einer die Wunde befeuchtenden Schicht zur Behandlung von Wunden während der Epithelisierungsphase eingesetzt wird.

Die Heilung von Hautwunden beruht auf der Fähigkeit der Haut Epithel- sowie Binde-und Stützgewebe zu regenerieren. Die Regeneration selbst ist durch ein komplexes Geschehen von ineinander übergreifenden Zellaktivitäten gekennzeichnet, die den Heilungsprozess schrittweise vorantreiben. So werden in der Literatur unabhängig von der Art der Wunde drei wesentliche Heilungsphasen einer Wunde beschrieben. Hierzu gehört die inflammatorische oder exsudative Phase zur Blutstillung und Wundreinigung (Phase 1, Reinigungsphase), die proliferative Phase zum Aufbau von Granulationsgewebe (Phase 2, Granulationsphase) und die Differenzierungsphase zur Epithelisierung und Narbenbildung (Phase 3, Epithelisierungsphase).

Unter einer Wunde wird die Trennung des Zusammenhangs von Geweben der Körperhülle bei Menschen oder Tieren verstanden. Sie kann mit einem Verlust an Substanz verbunden sein.

Zur Unterstützung der einzelnen Wundheilungsphasen sind zahlreiche Vorschläge in der Literatur beschrieben. So beschreibt beispielsweise die WO 2010/00451 eine mehrschichtige Wundauflage. Die Wundauflage enthält eine erste Schicht als Wundkontaktschicht, welche bevorzugt eine Hydrogelmatrix, einen Polymerfilm, eine Hydrokolloidmatrix, ein Polymernetz, einen Nonwoven (Vliesstoff) oder ein Adhäsiv umfasst und eine zweite absorbierende Schicht, die einen hydrophilen Polyurethanschaumstoff mit einem Wassergehalt von mindestens 10 Gew.-% und höchstens 80 Gew.-% Wasser umfasst. Darüber hinaus ist aus der WO 2011/141454 eine Wundauflage zur Wundbehandlung insbesondere im feuchten Milieu mit einem faservliesbasierten Saug-/Spülkörper bekannt, wobei in dem faservliesbasiertem Saug-/Spülkörper superabsorbierendes Material verteilt aufgenommen ist und dieser mit einer salzhaltigen wässrigen Lösung, insbesondere Ringerlösung, vorzugsweise bis zur Sättigung, beaufschlagt ist.

Die WO2014178945 beschreibt Systeme zur Wundbehandlung, die die Wundheilung mittels schwacher elektrischer Felder und/oder Mikroströmen positiv beeinflussen.

Der vorliegenden Erfindung liegt die Aufgabe zugrunde, die Wundbehandlung, insbesondere von Wunden in der Epithelisierungsphase, weiter zu verbessern. Es soll mit der vorliegenden Erfindung ein System zur Behandlung von Wunden zur Verfügung gestellt werden, mit dem eine Behandlung möglichst wirksam durchgeführt werden kann und welche beim Patienten nicht als unangenehm empfunden wird. Ferner soll das System vorteilhaft anwendbar sein.

Insbesondere soll die vorliegende Erfindung die Wundheilung in der Epithelisierungs-oder Regenerationsphase vorteilhaft beeinflussen, sodass beispielsweise eine kürzere Behandlungsdauer und/oder geringere Narbenbildung erreicht werden kann.

Die Aufgaben konnten unerwartet durch ein Wundsystem, welches zwei Schichten umfasst, gelöst werden, wobei die erste Schicht zwei räumlich voneinander getrennte Stoffe mit unterschiedlichem Standardpotential $E°$ enthält, sodass ein niedriger, elektrischer Mikrostrom erzeugt werden kann und wobei die zweite Schicht dazu geeignet ist, die Wunde zu befeuchten.

Ein Gegenstand der Erfindung ist daher ein Wundsystem, umfassend

(a) eine erste Schicht, enthaltend einen ersten und einen zweiten Stoff, wobei der erste und der zweite Stoff räumlich voneinander getrennt sind und gegebenenfalls leitend miteinander verbunden sind und wobei der erste und zweite Stoff ein unterschiedliches Standardpotential $E°$, gemessen bei 25°; 101,3 kPa, pH=0, Ionenaktivität = 1, aufweisen; und

(b) eine zweite, die Wunde befeuchtende Schicht, wobei die erste Schicht eine Wundkontaktschicht ist, welche im direkten Kontakt zur Wunde ist.

Beschrieben wird ein Stoffgemisch aus einem ersten und zweiten Stoff, wobei der erste und der zweite Stoff räumlich voneinander getrennt und gegebenenfalls leitend miteinander verbunden sind und die ein unterschiedliches Standardpotential $E°$ aufweisen, wobei das Stoffgemisch zusammen mit einer die Wunde befeuchtenden Schicht zur Behandlung von Wunden während der Epithelisierungsphase eingesetzt wird.

Das neue erfindungsgemäße Wundsystem zeichnet sich durch mehrere unerwartete Vorteile aus.

Durch die Kombination der Befeuchtung der Wunde und des Anlegens eines geringen elektrischen Mikrostroms, insbesondere während der Epithelisierungsphase, kann eine schnellere Wundheilung und/oder die vorteilhafte Verminderung der Narbenbildung erreicht werden. Das Anlegen eines geringen elektrischen Mikrostroms in Kombination mit der aus der zweiten Schicht bereit gestellten Befeuchtung scheint sich positiv auf die Wundheilung auszuwirken.

Die Bestandteile (a) und (b) des erfindungsgemäßen Wundsystems werden nachstehend beschrieben.

Das erfindungsgemäße Wundsystem umfasst eine erste Schicht, welche einen ersten und einen zweiten Stoff enthält, wobei der erste und der zweite Stoff räumlich voneinander getrennt sind und die ein unterschiedliches Standardpotential $E°$ aufweisen. In einer optionalen Ausführungsform sind der erste und der zweite Stoff leitend miteinander verbunden.

Die erste Schicht kann bevorzugt einen Schwamm, einen Film, einen Schaum, ein Gewebe, ein Gestrick, ein Filament, einen Vliesstoff (auch als "Nonwoven" bezeichnet), ein Netz, verwobene Materialien und /oder Kombinationen daraus umfassen.

Vorzugsweise kann die erste Schicht ein natürliches Material enthalten, wobei ein natürliches Material bevorzugt aus pflanzlicher oder tierischer Herkunft stammt. Beispiele für natürliche Materialien sind natürliche Schwämme, Polypeptide, Pektine, Seiden, Keratine, Alginate, und natürliche Fasern wie Baumwolle, Seide oder Zellstoff sowie Gemische davon.

Alternativ bevorzugt kann die erste Schicht ein synthetisches Material enthalten. Synthetische Materialien können auch natürlichen Ursprungs sein, wobei sie durch anschließende Behandlung, wie eine chemische Umsetzung, verändert werden. Beispiele sind Polymere wie Polyurethane, Polyolefine, Polyvinylalkohol, Poly(meth)acrylate, Acrylate, Kunstseide, Elastomere, künstliche Cellulosen wie Methylcellulose, Ethylcellulose und Hydroxypropylmethylcellulose, und künstliche Schwämme und Schäume sowie Gemische davon.

In einer bevorzugten Ausführungsform ist die erste Schicht flüssigkeitsdurchlässig. Unter flüssigkeitsdurchlässig soll verstanden werden, dass die Schicht einen Flüssigkeitsdurchgang von der einen Seite der ersten Schicht zur anderen Seite der ersten Schicht nicht vollständig unterbindet. In einer bevorzugten Ausführungsform kann die erste Schicht eine Vielzahl von Kanäle zum Durchtritt von Flüssigkeit umfassen. Insbesondere kann dabei vorgesehen sein, dass die erste Schicht Kanäle aufweist, mit welchen der Durchfluss von Flüssigkeit sowohl von der zweiten Schicht zum Wundgrund als auch umgekehrt gewährleistet wird.

Weiterhin enthält die erste Schicht einen ersten und einen zweiten Stoff, wobei der erste und der zweite Stoff räumlich voneinander getrennt sind, sowie ein unterschiedliches Standradpotential $E°$ aufweisen.

Eine galvanische Zelle kann als eine Vorrichtung zur Umwandlung von chemischer in elektrische Energie betrachtet werden, wobei die Wirkungsweise der galvanischen Zelle auf einer Redoxreaktion beruht. In der Regel umfasst die galvanische Zelle zwei Halbzellen (Halbelemente), in denen Oxidation und Reduktion räumlich getrennt voneinander ablaufen. Ein wesentlicher Bestandteil der galvanischen Zelle sind die Elektroden. Die Elektrode, an der die Oxidation abläuft, ist die Anode, von der die Elektronen über einen Leiter abfließen. Die Elektrode an der die Reduktion abläuft, ist die Kathode. Die Elektronen fließen über einen Leiter zur Kathode.

In einer bevorzugten Ausführungsform kann der erste und zweite Stoff, die räumlich voneinander getrennt, sowie ein jeweils unterschiedliches Standardpotential $E°$ aufweisen, als Elektroden betrachtet werden. In einer möglichen Ausführungsform sind der erste und der zweite Stoff leitend miteinander verbunden. Durch das unterschiedliche Standardpotential $E°$ der ersten und zweiten Substanz kann eine Spannung zwischen den Elektroden vorliegen. Gegebenenfalls kann auch ein elektrischer Strom fließen sobald die Elektroden durch einen Elektrolyten verbunden werden.

Der erste und der zweite Stoffe liegen räumlich getrennt voneinander vor. Der erste und zweite Stoff können in verschiedenen geometrischen Formen in der ersten Schicht enthalten sein. Die Grenzen dieser Formen sind jedoch klar definiert, sodass sie sich nicht überschneiden, sodass gewährleistet wird, dass der erste Stoff von dem zweiten Stoff räumlich getrennt ist.

In einer möglichen Ausführungsform sind der erste und der zweite Stoff leitend miteinander verbunden, bevorzugt durch einen Elektronenleiter. Die leitendende Verbindung kann beispielsweise in Form eines Drahtes sein, wenn die erste und die zweite Verbindung an genau zwei unterschiedlichen Stellen (Punkten) angeordnet werden. Alternativ kann die leitende Verbindung auch in Form eines Gitters (Arrays) vorliegen, wenn die erste und/oder die zweite Verbindung jeweils an mehreren Stellen (Punkten) angeordnet werden. Weiterhin ist die leitende Verbindung bevorzugt aus einem Metall oder einer metallischen Legierung. In einer bevorzugten Ausführungsform wird die leitende Verbindung von einem Isolator umgegeben, um einen möglichst verlustfreien Elektronentransport zwischen den Elektroden zu erreichen.

Der erste und der zweite Stoff weisen ein unterschiedliches Standardpotential $E°$ auf. Zwischen Halbzellen werden normalerweise nur Potentialdifferenzen gemessen. Daher wird dass Standardpotential einer Elektrode bzw. des Stoffes der Elektrode zu einer Bezugselektrode gemessen. Diese Bezugselektrode ist die Normal-Wasserstoffelektrode, welche aus Platin besteht, welche von Wasserstoff umspült wird und in eine Säurelösung mit der Konzentration von 1 mol/l, resultierend in einem pH von 0, taucht. Die Messung wird bei 101,3 kPa und 25°C durchgeführt. Das Potential der Normal-Wasserstoffelektrode wurde auf 0,00V festgelegt.

Die zwischen der Elektrode aus dem ersten Stoff und der Elektrode aus dem zweiten Stoff mögliche Spannung kann sich aus der Potentialdifferenz der Stoffe errechnen, nach

$$U = \Delta E = E_2 - E_1$$

wobei

$E_1$ das Standardpotential des ersten Stoffs (der ersten Elektrode) und
$E_2$ das Standardpotential der zweiten Stoffs (der zweiten Elektroden)

ist.

In einer bevorzugten Ausführungsform können der erste und zweite Stoff unabhängig voneinander an jeweils einer oder mehreren eigenständigen Stellen auf oder in der Schicht angeordnet sein. Sind mehrere solcher Stellen vorhanden, werden sie als Stellen für den ersten Stoff beziehungsweise Stellen für den zweiten Stoff bezeichnet.

Sowohl der erste als auch der zweite Stoff können bevorzugt auf der gleichen Oberflächenseite der ersten Schicht aufgebracht sein. In diesem Fall ist es eine mögliche Ausführungsform, dass auf die erste Schicht die leitende Verbindung aufgebracht wird und dann auf diese jeweils der erste und zweite Stoff.

Alternativ bevorzugt können der erste und zweite Stoff nahe zu einer Oberflächenseite in der ersten Schicht eingebracht sein. In einer möglichen Ausführungsform ist der erste und der zweite Stoff so dicht unter der Oberfläche eingebracht ist, dass trotzdem ein Kontakt mit dieser besteht, d.h. dass der der Oberfläche an nächsten liegende Punkt des ersten und zweiten Stoffs auf dem Niveau der Oberfläche ist, während die von der Oberfläche entfernteren Teile der Stelle des ersten und der Stelle des zweiten Stoffes weiter innerhalb der ersten Schicht liegen.

In einer bevorzugten Ausführungsform können sowohl die erste/n als auch die zweite/n Stelle/n unabhängig voneinander eine Fläche von 0,5 mm$^2$ bis 15mm$^2$, bevorzugt 0,7 mm$^2$ bis 10 mm$^2$, aufweisen. Alternativ im Falle von Nanolagerstellen kann die Fläche der Stelle/n für den ersten als auch den Stoff unabhängig 0,5 nm$^2$ bis 10000 nm$^2$ aufweisen Zudem können die Stelle/n für den ersten und denn zweiten Stoff bevorzugt eine dritte Dimension, wie Höhe oder Tiefe, aufweisen.

Die Stelle/n für den erste Stoff und Stelle/n für die zweite Substanz werden als ungleiche Stellen betrachtet. Bevorzugt sind eine oder mehrere Stellen für den ersten Stoff räumlich so angeordnet, dass sie von einer oder mehreren Stellen für den zweiten Stoff umgeben sein können.

In einer bevorzugten Ausführungsform ist eine eigenständige erste Lagerstelle benachbart zu einer oder mehreren zweiten Lagerstelle/n angeordnet. Benachbart kann in diesem Zusammenhang so verstanden werden, dass sie nahe beieinander sind, sodass, wenn ein leitendes Medium zwischen sie gebracht wird, ein elektrischer Strom fließen kann.

Durch die unterschiedliche Anordnung der Stelle/n für den ersten und den zweiten Stoff können unterschiedliche Muster (Pattern) gebildet werden, nach denen ein Stromfluss möglich ist.

In einer bevorzugten Ausführungsform sind der erste und der zweite Stoff jeweils ein Metall und/oder ein entsprechendes Metallsalz.

Beispiele für geeignete Metalle sind Gold (Au), Platin (Pt), Silber (Ag), Kupfer (Cu), Eisen (Fe), Zinn (Sn), Zink (Zn), Mangan (Mn), Titan (Ti), Aluminium (Al), Magnesium und Legierungen daraus.

Ein Metallsalz der oben aufgeführten beispielsweisen Metalle umfasst neben dem entsprechenden Metallkation ein oder mehrere Anionen. Beispiele für Anionen sind Halogenide, wie Fluorid, Chlorid, Bromid und Iodide, Oxide, Sulfide, Sulfate, Nitrate und Gemische daraus.

In einer bevorzugten Ausführungsform ist der erste Stoff Zink. Zink ist ein Metall, welches ein Standardpotential $E°$ von -0,76 Volt aufweist. Zink wird gewöhnlich als Reduktionsmittel eingesetzt, wobei Zink unter Elektronenabgabe zu $Zn^{2+}$ oxidiert wird. Zink zählt zu den essentiellen Spurenelementen für den menschlichen und tierischen Körper.

In einer bevorzugten Ausführungsform ist der zweite Stoff Silber und/oder ein Silbersalz. Beispiele für geeignete Silbersalze sind Silberhalogenide, wie Silberfluorid, Silberchlorid, Silberbromid, Silberiodid, Silberoxid und Gemische davon. Es ist besonders bevorzugt, dass der zweite Stoff eine Kombination aus Silber und dem entsprechenden Silbersalz ist. Silber ist ein Metall, welches ein Standardpotential $E°$ von 0,80 Volt aufweist. Für gewöhnlich werden Silberkationen als Oxidationsmittel eingesetzt, wobei sie unter Elektronenaufnahme zu Silber reduziert werden. Silber wirkt bakterizid und kann aus diesem Grund vorteilhaft im Rahmen einer Wundbehandlung/Wundheilung eingesetzt werden. Silberionen können ebenfalls in der Wundtherapie eingesetzt werden und wirken zudem desinfizierend. Weiterhin können Silber und Silberionen durch Hemmung des Bakterienwachstums unangenehme Gerüche verhindern.

Erfindungsgemäß ist die erste Schicht eine Wundkontaktschicht, d.h. sie ist im direkten Kontakt mit der Wunde. Weiterhin ist bevorzugt, dass die Oberflächenseite der ersten Schicht, auf welcher der erste und zweite Stoff aufgebracht sind oder in dessen Nähe der erste und der zweite Stoff eingebracht sind, direkten Kontakt zur Wunde hat.

In einer bevorzugten Ausführungsform hat die erste Schicht eine Dicke von 0,001 mm bis 5 cm, bevorzugt 0,1 mm bis 20 mm und ganz besonders bevorzugt von 0,25 mm bis 2,5 mm.

In einer bevorzugten Ausführungsform kann die zweite Schicht (b) eine Abstandschicht (b1) sowie eine Spen-

EP 3 040 086 B1

derschicht (b2) umfassen, wobei die Spenderschicht einen hydrophilen Polyurethanschaum mit mindestens 10 Gew.-% und höchstens 80 Gew.-% Wasser enthält.

In einer bevorzugten Ausführungsform umfasst die Spenderschicht (b2) einen hydrophilen Polyurethanschaum, der einen Wasseranteil von mindestens 20 Gew.-% Wasser, insbesondere mindestens 30 Gew.-% Wasser und ganz besonders bevorzugt mindestens 35 Gew.-% Wasser, umfasst. Hierbei ist weiterhin bevorzugt vorgesehen, dass der Polyurethanschaum einen Wasseranteil von höchstens 80 Gew.-% Wasser, insbesondere höchstens 70 Gew.-% und ganz besonders bevorzugt von höchstens 65 Gew.-% Wasser, umfasst.

Der Wasseranteil kann vorzugsweise in dem hydrophilen Polyurethanschaum homogen verteilt sein. Insbesondere umfasst der hydrophile Polyurethanschaum dabei einen Wasseranteil von mindestens 10 Gew.-% und höchstens 80 Gew.-% Wasser, wobei das Wasser insbesondere homogen in dem Polyurethanschaum verteilt ist.

Hierbei und im Folgenden soll im Zusammenhang mit der vorliegenden Erfindung - falls nichts anderes angegeben ist - jeder Gehalt eines Inhaltstoffes in Gewichtsprozent (Gew.-%), bezogen auf das Gewicht der den Inhaltsstoff umfassenden Komponente, verstanden sein.

Zur Überprüfung der Menge an Wasser einer Komponente soll im Zusammenhang mit der vorliegenden Erfindung die Norm DIN EN 14079 herangezogen werden, wobei die Menge an Wasser wie folgt berechnet wird:

$$W_w = \frac{W_g - W_t}{W_g} \bullet 100\% \qquad (1)$$

mit

$W_w$ = Gewicht des Wassers in %, bezogen auf das Gesamtgewicht der Komponente
$W_g$ = Gewicht der Wasser enthaltenden Komponente
$W_t$ = Gewicht der trockenen Komponente.

Damit soll im Zusammenhang mit der vorliegenden Erfindung unter einem Polyurethanschaum mit einem Wasseranteil von mindestens 10 Gew.-% ein solcher Polyurethanschaum verstanden sein, der mindestens 10 Gew.-% Wasser umfasst, wobei das Wasser von dem Polyurethanschaum freigesetzt werden kann. Im Unterschied hierzu ist nicht der Anteil an Wasser zu verstehen, der eventuell zur Bildung, beispielsweise bei der Polymerisation der Ausgangsprodukte des Polyurethanschaums, verwendet wird. Dieses Wasser wird kovalent gebunden und steht nicht der Wundbehandlung zur Verfügung. Darüber hinaus soll auch nicht ein Wasseranteil verstanden sein, der produktionsbedingt bei der Herstellung des Schaums verwendet wird. Dieser Wasseranteil wird nach oder während der Bildung des Polyurethanschaums dem Schaum meist durch Trocknen, beispielsweise durch Trocknen in einem Ofen, entzogen und steht somit auch nicht der Wundbehandlung zur Verfügung. Damit weist eine erfindungsgemäße Wundauflage einen Polyurethanschaum auf, der einen Wasseranteil umfasst, der deutlich einen eventuell durch die Herstellung nach Trocknung bedingten Restgehalt an Wasser übersteigt.

Weiterhin kann der hydrophile Polyurethanschaum einen Retentionswert R von mindestens 20 % aufweisen, bevorzugt mindestens 30 %, insbesondere mindestens 40 % und ganz besonders bevorzugt von mindestens 50 %, aufweisen.

Unabhängig hiervon kann weiterhin bevorzugt vorgesehen sein, dass der hydrophile Polyurethanschaum einen Retentionswert R von höchstens 90 %, insbesondere von höchstens 80 % und ganz besonders von höchstens 70 %, aufweist. Der Retentionswert R wird dabei gemäß der folgenden Methode bestimmt.

Der Retentionswert R beschreibt die Menge Wasser, die der Polyurethanschaum in seine Polyurethanmatrix maximal einbinden kann, wobei das Wasser, das in die Poren aufgenommen werden könnte, unberücksichtigt bleibt. Der Retentionswert wird bestimmt, indem ein Probenstück von 5 cm x 5 cm (unter Normklima gelagert) aus einem hydrophilen Polyurethanschaum mit einer Dicke von höchstens 5 mm ausgestanzt und dessen Gewicht unter Normklimabedingungen vermessen wird. Das Probenstück wird hiernach einer freien Absorption mit Wasser analog DIN EN 13726-1 unterworfen. Das Wasser, das von den Poren aufgenommen wurde, wird dem Probenstück mittels einer Rolle (Gewicht 5000 g, Durchmesser 10 cm, Breite 5 cm) herausgequetscht, indem die Probe mehrmals zwischen frische Zellstofftücher gelegt und mit der Rolle überrollt wird. Dieser Vorgang wird solange wiederholt, bis keine Wasserabsorption in den Zellstofftüchern mehr erkennbar ist. Zur Bestimmung des Retentionswertes R wird der Wasseranteil $W_{ww}$, der nach dem Absorbieren und dem Ausquetschen in dem Polyurethanschaum enthalten, ist gemäß DIN EN 14079 gemessen und wie folgt berechnet.

$$R = W_{ww} = \frac{W_{gg} - W_{tt}}{W_{gg}} \bullet 100\% = 52,8\ \%$$

**5**

(gemessen nach DIN EN 14079)
wobei gilt

$W_{ww}$ = das Gewicht des Wassers, das nach der Absorption und dem Ausquetschen in der Polyurethanschaum enthalten ist

$W_{tt}$ = das Gewicht des Probenstücks nach dem Trocknen ist, und

$W_{gg}$ = das Gewicht des Probenstücks nach Absorption und nach Ausquetschen ist.

Ganz besonders bevorzugt ist ein hydrophiler Polyurethanschaum, der einen Wasseranteil von mindestens 10 Gew.-% umfasst, wobei der Wasseranteil dem Retentionswert R des Polyurethanschaums entspricht.

Die Wunde sowie der erste und der zweite Stoff werden von Anfang an befeuchtet oder mit Feuchtigkeit versorgt, wobei die Spenderschicht gleichzeitig durch den Polyurethanschaum eine ausreichende Absorptionskapazität aufweist. Hierdurch werden die Wundheilung eventuell negativ beeinflussende Faktoren der Wundoberfläche entzogen und gleichzeitig Feuchtigkeit und Wasser in einer ausreichenden Menge zur Verfügung gestellt.

Aufgrund der etwas geringeren Absorptionskapazität gegenüber trockenen Polyurethanschäumen, die kein freisetzbares Wasser umfassen und meist sehr schnell sehr viel Wundsekret aufnehmen, wodurch eine trockene Wundoberfläche gebildet wird, ist diese zweite Schicht hervorragend geeignet in der Epithelisierungs- oder Granulationsphase, insbesondere der Epithelisierungsphase der Wundheilung, eingesetzt zu werden. Damit kann der hydrophile Polyurethanschaum in natürlicher Weise die Granulation und/ oder die Epithelisierung der Wunde in besonderem Maße fördern.

In einer bevorzugten Ausführungsform kann der hydrophile Polyurethanschaum einen Wasseranteil von mindestens 10 Gew.-% und ein Quellvermögen $\Delta V_1$ von höchstens 80 % aufweisen. Insbesondere weist der hydrophile Polyurethanschaum dabei ein Quellvermögen $\Delta V_1$ von höchstens 60 %, insbesondere von höchstens 40 %, insbesondere von höchstens 30 %, und ganz besonders bevorzugt von höchstens 20 %, auf. Dabei kann weiterhin vorteilhaft vorgesehen sein, dass der Polyurethanschaum ein Restquellvermögen $\Delta V_1$ von mindestens 5 % aufweist. Dieses Restquellvermögen kann ausgenützt werden damit die Wundauflage während der Absorption einen besseren Kontakt zum Wundgrund annehmen kann.

Hierbei soll unter dem Quellvermögen $\Delta V_1$ eines Polyurethanschaums die Volumenzunahme verstanden sein, die ein Polyurethanschaum, der seine Absorptionskapazität vollständig erschöpft hat, im Vergleich zu einem Polymerurethanschaum mit einem Wassergehalt von mindestens 10 Gew.-% Wasser, erfährt. Das Quellvermögen soll dabei gemäß einem hierin beschriebenen Test ermittelt werden.

Das Quellvermögen $\Delta V_1$ eines Polyurethanschaums beschreibt die Volumenänderung, die ein wasserhaltiger Polyurethanschaum erfährt, nachdem er seine maximale Absorption erreicht hat. Zur Bestimmung des Quellvermögens $\Delta V_1$ werden die räumlichen Abmessungen eines Probenstücks des wasserhaltigen Polymerschaums und die räumlichen Abmessungen dieses Probenstücks nach vollständiger Absorption gemäß der freien Absorption nach DIN EN 13726-1 bestimmt. Zur Bestimmung der Dicke (Höhe) wird ein Dickenmessgerät mit 25 cm$^2$ Teller verwendet, wobei eine Belastung von 2 g/cm$^2$ gemäß EN ISO 9073-2 eingestellt wird. Die laterale Ausdehnung (Länge, Breite) wird mittels einer Schieblehre bestimmt, ohne dass das Probenstück deformiert wird. Zur Bestimmung der Ausdehnung wird das jeweilige Probenstück spannungsfrei auf eine glatte Oberfläche abgelegt. Die Volumenänderung nach Absorption entspricht dem Quellvermögen $\Delta V_1$ des wasserhaltigen Polyurethanschaums, wobei alle drei Raumrichtungen beachtet werden.

$$\Delta V_1 = \frac{V_2 - V_1}{V_1} \bullet 100\% = \frac{(l_2 \bullet b_2 \bullet h_2) - (l_1 \bullet b_1 \bullet h_1)}{(l_1 \bullet b_1 \bullet h_1)} \bullet 100\%$$

wobei gilt

$V_1$ = das Volumen des wasserhaltigen Probenstücks ist, und

$V_2$ = das Volumen des Probenstücks nach vollständiger Absorption ist.

Als Polyurethanschaum kann im Zusammenhang mit der vorliegenden Erfindung jeder heute in der modernen Wundbehandlung übliche hydrophile Polyurethanschaum verwendet werden, der einen Wasseranteil in seine Polyurethanmatrix aufnimmt und eine ausreichende Absorption aufweist. Damit ist im Zusammenhang mit der vorliegenden Erfindung unter einem hydrophilen Polyurethanschaum ein solcher Polyurethanschaum zu verstehen, der eine Flüssigkeit in seine Polyurethanmatrix und in seine Poren aufnehmen und speichern, somit absorbieren, kann und zumindest einen Teil der aufgenommenen Flüssigkeit wieder abgeben kann. Als hydrophile Polymerschäume sind hierbei insbesondere offenporige, hydrophile Polyurethanschäume geeignet. Demgemäß umfasst eine besonders bevorzugte Wundauflage eine zweite Schicht, die einen offenporigen, hydrophilen Polyurethanschaum umfasst.

Bevorzugt sollen solche Polyurethanschäume eingesetzt werden, die eine hohe Absorptionskapazität aufweisen. Diese Absorptionskapazität soll vorhanden sein, obwohl der Polyurethanschaum einen Anteil seines Eigengewichts an Wasser in seine Polymermatrix aufgenommenen hat. Bevorzugt ist ein Polyurethanschaum, der einen Wasseranteil von mindestens 10 Gew.-% und höchstens 80 Gew.-% umfasst und der eine freie Absorption $A_2$ von mindestens 10 g/g, insbesondere mindestens 12 g/g und ganz besonders bevorzugt von mindestens 15 g/g aufweist, wobei die freie Absorption $A_2$ gemäß der DIN-EN 13726-1 (2002) bestimmt wird. Hierbei ist die freie Absorption $A_2$ die freie Absorption des Wasser enthaltenden Polyurethanschaums.

Weiterhin bevorzugt kann der hydrophile Polyurethanschaum eine durchschnittliche Porengröße von weniger als 1000 $\mu$m, insbesondere 100 $\mu$m bis 1000 $\mu$m, insbesondere 100 $\mu$m bis 500 $\mu$m und ganz besonders bevorzugt 100 $\mu$m bis 300 $\mu$m, aufweisen. Hierbei kann insbesondere vorgesehen sein, dass die durchschnittliche Porengröße an der ersten Seite der zweiten Schicht gleich groß ist wie die Porengröße im Inneren der zweiten Schicht und/ oder gleich groß ist wie an der zweiten Seite der zweiten Schicht. Weiterhin bevorzugte hydrophile Polyurethanschäume weisen eine Dichte von weniger als 150 kg/m$^3$, insbesondere weniger als 140 kg/m$^3$ und ganz besonders bevorzugt 50 kg/m$^3$ bis 120 kg/m$^3$ auf. Die Bestimmung der Porengröße erfolgt bevorzugt mikroskopisch, wobei ein Probenquerschnitt mikroskopiert wurde; die angegebene Porengröße entspricht dem Mittelwert von 5 zufällig ausgewählten und ausgemessenen Poren pro Probe.

Als besonders vorteilhafte Ausführungsform hat sich gezeigt, dass die Spenderschicht eine Dicke von 0,1 bis 5,0 mm insbesondere von 0,5 bis 5,0 mm und ganz besonders bevorzugt von 0,5 bis 3,0 mm aufweist. Solche Schichtdicken zeigen einerseits die Fähigkeit auf, ein von einer Wunde abgegebenes Wundexsudat aufzunehmen und gleichzeitig eine ausreichende Menge an Wasser oder Feuchtigkeit einer Wunde bereitstellen zu können. Diese Schichtdicken können an jeder Stelle gleich sein oder in verschiedenen Bereichen verschiedene Werte annehmen. Insbesondere ist auch vorgesehen, dass die Spenderschicht abgeflachte Ränder aufweist.

Als Abstandschicht (b1) kann gemäß der vorliegenden Erfindung ein Material Verwendung finden, das keinen negativen Einfluss auf die Wundheilung ausübt. Hierbei bevorzugt steht die Abstandsschicht in direktem Kontakt zur ersten Schicht (a).

In einer bevorzugten Ausführungsform kann die Abstandschicht eine Hydrogelmatrix, ein Polymerfilm, eine Hydrokolloidmatrix, ein Polymernetz, ein Nonwoven, ein Adhäsiv oder eine Polymernetz umfassen. Insbesondere bevorzugt sind ein Polyurethanfilm oder ein Polymernetz.

Gemäß einer weiteren Ausführungsform der vorliegenden Erfindung kann weiterhin vorgesehen sein, dass die Abstandsschicht eine Hydrokolloidmatrix umfasst. Diese Hydrokolloidmatrix kann aus einer klebenden Polymermatrix bestehen, in die Hydrokolloidpartikel dispergiert sind. Gemäß der vorliegenden Erfindung soll unter einem Hydrokolloid ein Material verstanden sein, das ein hydrophiles synthetisches oder natürliches Polymermaterial ist, das löslich oder absorbierend und/ oder quellend in Wasser ist. Vorzugsweise umfasst eine Wundkontaktschicht ein Hydrokolloid aus einem synthetischen oder natürlichen Polymermaterial, das ausgewählt wird aus der Gruppe Alginsäure und deren Salze sowie deren Derivate, Chitin oder dessen Derivate, Chitosan oder dessen Derivate, Pektin, Cellulose oder dessen Derivate, wie Celluloseether oder Celluloseester, vernetzte oder nicht vernetzte Carboxyalkylcellulose oder Hydroxyalkylcellulose, Polyvinylalkohol, Polyvinylpyrrolidon, Agar, Guargummi, Gelatine oder Mischungen hiervon.

Das Hydrokolloid kann sowohl in Form von Fasern als auch in Form von Partikeln und/oder Fasern innerhalb einer Matrix vorliegen. Insbesondere kann das Hydrokolloid in Form von Partikeln in einer klebenden Polymermatrix vorliegen. Die klebende Polymermatrix umfasst dabei mindestens ein Co-Blockpolymer, ausgewählt aus der Gruppe der AB-Diblock-Copolymere und/ oder ABA-Triblock-Copolymere, die aus den Monomeren Styrol, Butadien und Isopren aufgebaut sind. Der Anteil an Hydrokolloidpartikel in der Abstandsschicht kann vorzugsweise 10 bis 70 Gew.-%, bezogen auf das Gesamtgewicht der zweiten Schicht, sein.

Gemäß einer weiteren Ausführungsform der vorliegenden Erfindung kann weiterhin vorgesehen sein, dass die Abstandsschicht eine wasserhaltige Hydrogelmatrix umfasst. Diese Hydrogelmatrix kann vorzugsweise mindestens 20 Gew.-%, insbesondere mindestens 30 Gew.-%, insbesondere mindestens 40 Gew.-% und ganz besonders bevorzugt mindestens 50 Gew.-% Wasser umfassen, wobei die Hydrogelmatrix weiterhin bevorzugt höchstens 90 Gew.-% und weiterhin bevorzugt höchsten 80 Gew.- % Wasser umfasst. Somit kann eine zweite Schicht bereitgestellt werden, die eine Abstandsschicht aufweist, die neben der weiter unten beschriebenen Spenderschicht eine weitere wasserhaltige Komponente enthält und somit eine gewisse Menge an Feuchtigkeit zur Verfügung stellt.

Als wasserhaltige Hydrogelmatrices können im Zusammenhang mit der vorliegenden Erfindung insbesondere Hydrogelmatrices verwendet werden, die eine zusammenhängende, diskrete Schicht bilden und unter Druck kein Wasser abgeben. Insbesondere sind im Zusammenhang mit der vorliegenden Erfindung Hydrogelmatrices geeignet, die ein Polyurethan-Polyharnstoff-Copolymer umfassen. Dieses Polyurethan-Polyharnstoff-Copolymer kann dabei insbesondere aus einem Präpolymer mit aliphatischen Diisocyanat-Gruppen und einem Polyamin auf Polyethylenoxidbasis gebildet werden. Insbesondere kann das Polyurethan-Polyharnstoff-Copolymer dabei aus einem Präpolymer mit Isophorondiisocyanat-Enden, einem Polyamin auf Polyethylenoxidbasis und Wasser gebildet werden. Diese Hydrogelmatrices sind besonders gut geeignet Wasser einzulagern und dieses Wasser an eine Wunde abzugeben.

Weiterhin bevorzugt kann die wasserhaltige Hydrogelmatrix weiterhin mindestens einen mehrwertigen Alkohol aus der Gruppe der zweiwertigen, dreiwertigen, vierwertigen, fünfwertigen oder sechswertigen Alkohole umfassen. Insbesondere kann der Alkohol gewählt werden aus der Gruppe der Glykole, insbesondere Ethylenglykol oder Propylenglykol, sowie Sorbitol oder Glycerin oder Mischungen hiervon. Diese Alkohole sind hervorragend als Feuchtigkeitsspender geeignet und stellen somit für die die Wunde umgebende Haut eine pflegende Komponente dar.

Dabei kann die wasserhaltige Hydrogelmatrix insbesondere 0 bis 50 Gew.-% eines mehrwertigen Alkohols umfassen. Insbesondere umfasst die Hydrogelmatrix 5 bis 40 Gew.-% eines mehrwertigen Alkohols und ganz besonders bevorzugt 10 bis 30 Gew.-% eines mehrwertigen Alkohols.

Darüber hinaus kann vorgesehen sein, dass die wasserhaltige Hydrogelmatrix insbesondere mindestens ein Salz umfasst. Insbesondere ist hierbei vorgesehen, dass die Hydrogelmatrix ein anorganisches Salz umfasst. Besonders geeignet sind in diesem Zusammenhang Chloride, Iodide, Sulfate, Hydrogensulfate, Karbonate, Hydrogenkarbonate, Phosphate, Dihydrogenphosphate oder Hydrogenphosphate der Alkali- und Erdalkalimetalle. Ganz besonders bevorzugt umfasst die Hydrogelmatrix Natriumchlorid, Kaliumchlorid, Magnesiumchlorid, Kalziumchlorid oder Mischungen hiervon. Diese Salze simulieren in besonders guter Weise das Elektrolyt-Gemisch in einem von einer Wunde abgegebenen Wundserum. Damit stellt eine diese Salze umfassende Hydrogelmatrix einer Wunde ein besonders wundheilungsförderndes Klima zur Verfügung.

Hierbei kann vorgesehen sein, dass die Hydrogelmatrix 0 bis 5 Gew.-% mindestens eines Salzes umfasst. Insbesondere umfasst die Hydrogelmatrix 0,1 bis 3 Gew.-% eines Salzes und ganz besonders bevorzugt 0,5 bis 1,5 Gew.-% eines Salzes.

Insgesamt kann gemäß der vorliegenden Erfindung vorgesehen sein, dass die wasserhaltige Hydrogelmatrix bevorzugt mindestens 20 Gew.-% Wasser und mindestens 10 Gew.-% Polyurethan-Polyharnstoff-Copolymer umfasst. Eine weiterhin bevorzugte Hydrogelmatrix umfasst mindestens 20 Gew.-% Wasser und mindestens 15 Gew.-% Polyurethan-Polyharnstoff-Copolymer. Hierbei kann weiterhin bevorzugt vorgesehen sein, dass die Hydrogelmatrix aus 6 bis 60 Gew.-% eines Präpolymers mit aliphatischen Diisocyanat-Gruppen, 4 bis 40 Gew.-% Polyamin auf Polyethylenoxidbasis, 0 bis 50 Gew.-% eines mehrwertigen Alkohols, 0 bis 5 Gew.-% mindestens eines Salzes, ausgewählt aus der Gruppe Natriumchlorid, Kaliumchlorid, Magnesiumchlorid, Kalziumchlorid oder Mischungen hiervon, und mindestens 20 Gew.-% Wasser gebildet wird.

Weiterhin bevorzugt kann vorgesehen sein, dass die Hydrogelmatrix aus 6 bis 30 Gew.- % eines Präpolymers mit aliphatischen Diisocyanat-Gruppen, 4 bis 20 Gew.-% Diamin auf Polyethylenoxidbasis, 10 bis 30 Gew.-% eines mehrwertigen Alkohols, ausgewählt aus der Gruppe bestehend aus Propylenglycol und/ oder Glycerin, 0,5 bis 1,5 Gew.-% eines Salzes, ausgewählt aus der Gruppe Natriumchlorid, Kaliumchlorid, Magnesiumchlorid, Kalziumchlorid oder Mischungen hiervon, und mindestens 30 Gew.-% Wasser gebildet wird.

Ganz besonders bevorzugt kann vorgesehen sein, dass die Hydrogelmatrix aus 6 bis 20 Gew.-% Präpolymer mit Isophorondiisocyanat-Enden, 4 bis 15 Gew.-% Diamin auf Polyethylenoxidbasis, 15 bis 20 Gew.-% Polypropylenglycol und/ oder Glycerin, 0,5 bis 1,5 Gew.-% eines Salzes, ausgewählt aus der Gruppe Natriumchlorid, Kaliumchlorid, Magnesiumchlorid, Kalziumchlorid oder Mischungen hiervon, und mindestens 40 Gew.-% Wasser gebildet wird. Diese Hydrogelmatrix weist eine freie Absorption $A_3$ (gemessen nach DIN EN 13723-1 (2002)) von mindestens 1 g/g und höchstens 5 g/g auf, stellt ein nicht reizendes, flüssigkeitsabsorbierendes, vor Bakterien schützendes, polsterndes, hautartiges Medium bereit und ist somit besonders gut als Wundkontaktschicht geeignet.

Als besonders vorteilhafte Ausführungen haben sich weiterhin Abstandsschichten gezeigt, die eine Hydrogelmatrix umfassen, deren Schichtdicke 0,1 mm bis 5,0 mm, bevorzugt 0,5 mm bis 5,0 mm und ganz besonders bevorzugt von 0,5 mm bis 3,0 mm beträgt. Diese Schichtdicken können an jeder Stelle der Abstandsschicht gleich sein oder in verschiedenen Bereichen der Abstandschicht verschiedene Werte annehmen.

Weiterhin bevorzugt kann die Hydrogelmatrix Kanäle umfassen, insbesondere konische Kanäle, zum Durchtritt von Flüssigkeiten von der ersten Schicht zur Spenderschicht. Hierbei ist besonders bevorzugt vorgesehen, dass die Kanäle einen elliptischen oder einen kreisförmigen Querschnitt aufweisen, d.h. dass die Kanäle eine kreisförmige oder elliptische Öffnung sowohl an der ersten als auch an der zweiten Seite der Hydrogelmatrix aufweisen, wobei die kreisförmige oder elliptische Öffnung auf der ersten und der zweiten Seite verschieden groß ist. Es kann jedoch auch vorgesehen sein, dass die Kanäle einen dreieckigen, rechteckigen, quadratischen, fünfeckigen, sechseckigen oder einen anderen vieleckigen Querschnitt aufweisen.

Gemäß einer Ausführungsform der Erfindung kann auch vorgesehen sein, dass die Abstandsschicht Öffnungen aufweist, die einen Durchmesser von 0,5 mm bis 5 mm aufweisen. Insbesondere weist die Abstandschicht Öffnungen auf, die einen Durchmesser von 1 mm bis 3 mm aufweisen. Ganz besonders bevorzugt weist die Abstandsschicht auf der der ersten Schicht zugewandten Seite Öffnungen auf, die einen Durchmesser von 1 mm bis 3 mm aufweisen, wobei die andere Seite der Abstandsschicht in direktem Kontakt mit dem Polyurethanschaum steht.

Es kann jedoch auch vorgesehen sein, dass zwischen der Abstandschicht und der Spenderschicht eine Übergangsschicht angeordnet ist. In dieser Ausführungsform kann die zweite Schicht zwischen der Abstandsschicht und der Spenderschicht eine Übergangsschicht aufweisen, die beide Materialen umfasst. Diese Übergangsschicht kann ebenso wie die Wundkontaktschicht Kanäle, Öffnungen oder Löcher aufweisen. Falls die Übergangsschicht Kanäle, Öffnungen oder Löcher aufweist, sind gemäß einer weiter bevorzugten Ausführungsform diese Kanäle, Öffnungen oder Löcher mit Polyurethanschaum ausgefüllt. Weiterhin bevorzugt sind diese Kanäle, Öffnungen oder Löcher kongruent zu den Kanälen, Öffnungen oder Löchern der Wundkontaktschicht. Durch die Anordnung einer solchen Übergangsschicht kann eine zweite Schicht bereitgestellt werden, die ein Laminat aus Abstandsschicht und Spenderschicht umfasst, wobei das Laminat einen besonders festen Zusammenhalt zwischen der Abstandsschicht und der Spenderschicht aufweist.

Gemäß einem weiteren weiterführenden Gedanken der vorliegenden Erfindung kann die zweite Schicht auch eine Barriereschicht zwischen Abstandsschicht und Spenderschicht aufweisen. Eine solche Barriereschicht kann beispielsweise einen Polymerfilm umfassen, der mit Öffnungen versehen ist.

Weiterhin kann die zweite Schicht eine Trägerschicht umfassen. Diese Trägerschicht kann aus verschiedenen Materialien, wie Nonwoven, Polymerfilm oder Polymerschaum aufgebaut sein. Diese Trägerschicht kann direkten Kontakt oder indirekten Kontakt zu der zweiten Seite der Spenderschicht aufweisen. Bei einem direkten Kontakt wird die Trägerschicht direkt auf die Spenderschicht auflaminiert, wogegen bei einem indirekten Kontakt die Trägerschicht mittels eines Adhäsivs auf die Spenderschicht aufgebracht ist. Dabei kann das Adhäsiv vollflächig oder lediglich in Teilbereichen zwischen der Trägerschicht und der Spenderschicht aufgebracht sein.

Als Trägerschicht können insbesondere Polymerfilme oder Polymerschäume eingesetzt werden. Ganz besonders bevorzugt sind Polymerfilme oder Polymerschäume, die wasserundurchlässig sind und die eine hohe Wasserdampfdurchlässigkeit aufweisen. Hierzu sind besonders Filme oder Schäume geeignet, die aus Polyurethan, Polyetherurethan, Polyesterurethan, Polyether-Polyamid-Copolymeren, Polyacrylat oder Polymethacrylat gefertigt werden. Insbesondere ist als Trägerschicht ein wasserundurchlässiger und wasserdampfdurchlässiger Polyurethanfilm oder ein wasserundurchlässiger und wasserdampfdurchlässiger Polyurethanschaum geeignet. Insbesondere ist als Polymerfilm ein Polyurethanfilm, Polyesterurethanfilm oder Polyetherurethanfilm bevorzugt. Ganz besonders sind aber auch solche Polymerfilme bevorzugt, die eine Dicke von 15 $\mu$m bis 50 $\mu$m, insbesondere 20 $\mu$m bis 40 $\mu$m und ganz besonders bevorzugt von 25 $\mu$m bis 30 $\mu$m aufweisen. Die Wasserdampfdurchlässigkeit des Polymerfilms der Wundauflage weist vorzugsweise mindestens 750 g/m$^2$/24 Std., insbesondere mindestens 1000 g/m$^2$/24 Std. und ganz besonders bevorzugt mindestens 2000 g/m$^2$/24 Std. auf (gemessen nach DIN EN 13726). In besonders bevorzugten Ausführungsformen weisen diese Filme einen feuchtigkeitsdichten, klebenden Randabschnitt auf. Dieser Randabschnitt gewährleistet, dass das Wundsystem an seinem bestimmungsgemäßen Ort appliziert und fixiert werden kann. Darüber hinaus ist sichergestellt, dass keine Flüssigkeit zwischen der Folie und der die zu behandelnden Fläche umgebende Haut austreten kann. Als besonders bevorzugt sind solche Klebstoffe zu betrachten, die in einem dünnen Auftrag von 20 g/m$^2$ bis 35 g/m$^2$ zusammen mit dem Film eine Wasserdampfdurchlässigkeit von mindestens 800 g/m$^2$/24 Std. und vorzugsweise von mindestens 1000 g/m$^2$/24 Std. (gemessen nach DIN EN 13726) aufweisen.

In einer alternativ bevorzugten Ausführungsform kann eine zweite die Wunde befeuchtende Schicht (b) einen faservliesbasierten Saug/Spülkörper (c), in dem superabsorbierendes Material verteilt aufgenommen ist und der mit einer salzhaltigen Lösung beaufschlagt ist, umfassen.

Bei der salzhaltigen Lösung handelt es sich bevorzugt um eine salzhaltige wässrige Lösung. Geeignete Salze zum Fertigen der Lösung sind alle physiologisch verträglichen Salze. Geeignete Salze sind beispielsweise Natriumchlorid NaCl, Kaliumchlorid KCl, Kaliumiodid KI, Kaliumsulfat K$_2$SO$_4$, Magnesiumsulfat MgSO$_4$, Calciumchlorid CaCl$_2$, Eisenphosphat FePO$_4$ und Gemische davon.

Bevorzugt handelt es sich bei der wässrigen Salzlösung um eine Ringerlösung, welche typischerweise Natriumchlorid, Kaliumchlorid und Calciumchlorid , insbesondere 8,6 g NaCl, 0,3 g KCl und 0,33 g CaCl$_2$ je Liter enthält.

Bevorzugt kann die wässrige Lösung eine bei pH-Werten von 4 bis 7,5 eines typischen feuchten oder feuchtnassen Wundmilieus kationische Substanz mit antimikrobieller Wirkung umfassen. Diese Substanz kann bevorzugt von negativen Gruppen des superabsorbierenden Materials, bevorzugt anionischen superabsorbierenden Materials, attraktiv angezogen werden und so innerhalb des Saug-/Spülkörpers antimikrobiell wirken. Somit kann eine hervorragende Konditionierung des Saug-/Spülkörpers (c) auch für Anwendungen über 24 Stunden hinaus realisiert werden, was sich wiederum vorteilhaft auf die Konditionierung des Wundmilieus auswirkt, da im Flüssigkeitsaustausch im Betrieb des Saug-/Spülkörpers Flüssigkeit aus dem antimikrobiell optimal konditionierten Saug-/Spülkörper zur Wunde gelangt, jedoch im Wesentlichen ohne dass hierbei septisch wirkende Substanzen oder Keime zurück zur Wunde gelangen. Es wird also die Rückkontamination weitestgehend verhindert.

Bei der kationischen Substanz mit antimikrobieller Wirkung kann es sich beispielsweise um Substanzen mit Amino- oder Iminogruppen handeln, welche in einer Lösung mit einem pH-Wert von 4 bis 7,5 kationisch geladen vorliegen. Weiterhin kann es sich bei der kationischen Substanz um antimikrobiell wirksame Metallkationen handeln, insbesondere um Silberkationen, beispielsweise um einen Komplex von 1-Vinyl-2-Pyrrolidon mit Silber-Kationen. Besonders geeignete kationischen Substanzen mit antimikrobieller Wirkung sind Biguanid-Derivate wie Chlorhexidin oder Polybiguanide, wie Polyethylene Biguanid (PEB), Polytetramethylenbiguanid (PTMB) oder Polyethylenhexamethylenbiguanide (PEHMB). Eine besonders bevorzugtes Polybiguanid ist Polyhexamethylenbiguanid (PHMB, bzw. Polyhexanid). Weitere geeignete kationischen Substanzen mit antimikrobieller Wirkung sind Polyguanidine wie zum Beispiel Polyhexamethylenguanidine (PHMG), N-Octyl-1-[10-(4-Octyliminopyridin- 1-yl)decyl]pyridin-4-imin (Octenidin), quartäre Ammoniumverbindungen, wie zum Beispiel Benzalkoniumchlorid oder Cetylpyridiniumchlorid, Triazine wie zum Beispiel 1-(3-Chloroallyl)-3,5,7-Triaza-1-Azoniaadamantan-Chlorid oder die Ammoniumverbindung Taurolidin.

Die Konzentration des antiseptisch wirkenden Zusatzes in der wässrigen Lösung, die dann zur Aktivierung des trockenen Saug/Spülkörpers verwendet wird, beträgt vorzugsweise 0,06 bis 0,20 Gew.-%, insbesondere 0,08 bis 0,15 Gew.-%, insbesondere 0,10 bis 0,15 Gew.-% (bezogen auf die wässrige Lösung vor dem Aktivieren des Saug/Spülkörpers).

Wie oben bereits ausgeführt, wird durch die Wahl der Substanz mit antimikrobieller Wirkung im Zusammenwirken mit dem superabsorbierenden Material erreicht, dass diese Substanz auch im Betrieb der Wundauflage weitgehend oder weitestgehend innerhalb des Saug-/Spülkörpers verbleibt. Dies kann anhand des Hemmhoftests (Englisch: zone of inhibition test) visuell geprüft bzw. verifiziert werden. Bei der Durchführung des nachfolgend im Einzelnen beschriebenen Hemmhoftests kann visuell überprüft werden, ob aus einem Testkörper keimtötende Substanzen in wesentlicher Menge austreten oder nicht. Treten sie aus, so töten oder behindern sie das Wachstum sogenannter koloniebildender Einheiten in einer Umgebung des Testkörpers, was visuell durch Inaugenscheinnahme des Testkörpers und seines Umfelds festgestellt werden kann. Lässt sich keine solche Umgebung, also kein Hemmhof (oder englisch: zone of inhibition) feststellen, so bedeutet dies, dass aus dem Testkörper keine das Koloniewachstum in nennenswertem Umfang beeinträchtigende Substanzen ausgetreten sind. Zur noch weitergehenden Charakterisierung des Gegenstands der vorliegenden Erfindung ist die Wundauflage weiter dadurch gekennzeichnet, dass bei Ausführung des Hemmhoftests ein Austreten von kationischer Substanz mit antimikrobieller Wirkung aus der Wundauflage visuell nicht feststellbar ist. Dies bedeutet, dass ein Hemmhof bei Ausführung des nachfolgend beschriebenen Tests visuell nicht feststellbar ist.

Zur Durchführung des Hemmhoftests wird eine Wundauflage der hier in Rede stehenden Art als Testkörper auf eine zuvor vorbereitete Agarplatte aufgelegt. Es wird hierfür eine Agarplatte mit einem Durchmesser von 8,5 cm eingesetzt, die jeweils 15 ml Caseinpepton-Sojapepton-Agar enthielt (Konzentration des Caseinpepton-Sojapepton: 40 g/l). Auf diese Agarplatte wurde 100 $\mu$l einer Staphylokokkus aureus ATCC 6538 Keimsuspension (etwa $5 \times 10^6$ koloniebildende Einheiten/ml) mit einem Wattestäbchen aufgestrichen und für 10 bis 15 Minuten getrocknet. Nach dem Trocknen der Keimsuspension bei geschlossenem Deckel wird der sterilisierte, jedoch auf Raumtemperatur abgekühlte Testkörper in Form der zu testenden Wundauflage auf die Agarplatte mit der wundzugewandten Seite aufgelegt. Die Platte wird dann aufrecht stehend für 18 Stunden bei 35 °C inkubiert. Danach wird die Agarschale visuell nach der Bildung einer Hemmzone untersucht. Die Hemmzone wird typischerweise in Millimeter nach der Formel H = (D - d) : 2 berechnet, wobei D der Gesamtdurchmesser von Testkörper und Hemmzone und d der Durchmesser des Testkörpers, jeweils in Millimeter, ist. Sofern eine Hemmzone um den Testkörper herum visuell nicht feststellbar ist (D = d und H = 0), so ist der Austritt etwaiger Substanzen aus dem Testkörper und deren Auswirkung auf das Koloniewachstum nach dem Hemmhoftest nicht feststellbar.

In einer bevorzugten Ausführungsform kann der faservliesbasierte Saug/Spülköper auf der zur ersten Schicht zugewandten Seite eine partiell und strukturiert aufgebrachte Beschichtung aufweisen. So kann vorzugweise ein besserer Flüssigkeitsaustausch über die beschichtungsfreien Bereiche erreicht werden. Dies führt zu einem insgesamt besseren Flüssigkeitsaustausch, der sich wiederum im Zusammenwirken mit der kationischen Substanz mit antimikrobieller Wirkung im Inneren des Saug-/Spülkörpers förderlich auf die Wundheilung auswirkt, da Wundsekrete/-exsudate mit Keimen in größerem Umfang in den Saug-/Spülkörper gelangen können und dort antimikrobiell behandelt werden können. Der Begriff der partiell und strukturiert aufgebrachten Beschichtung impliziert, dass es sich hierbei nicht um eine flächenhaft durchgehende Beschichtung handelt, sondern um eine poröse Beschichtung, wie sie beispielsweise durch Punkte, Inseln, Linien, Streifen oder sonstige zusammenhängende oder nicht zusammenhängende Strukturen realisiert werden kann.

Weiter erweist sich ein Bedeckungsgrad der partiell und strukturiert aufgebrachten wirkenden Beschichtung von 40 bis 70 %, insbesondere von 40 bis 60 % und weiter insbesondere von 40 bis 55 Gew.-%, insbesondere 40 bis 50 % der Fläche der betrachteten ersten Schicht zugewandten Umhüllung als vorteilhaft.

Die partiell und strukturiert aufgebrachte wirkende Beschichtung hat vorzugsweise eine Abmessung in wenigstens einer Ebenenrichtung von höchstens 4 mm, insbesondere von höchstens 3 mm, und weiter insbesondere von 1 bis 3 mm, insbesondere von 2 bis 3 mm.

Vorzugsweise handelt es sich bei dem superabsorbierenden Material, bevorzugt bei dem anionischen, superabsorbierenden Material, um ein Polymer, insbesondere um ein zumindest partiell quervernetztes Polymer, insbesondere auf Polyacrylatbasis. Gemäß einer weiteren Ausführungsform handelt es sich bei dem anionischen superabsorbierenden Material um ein Polysaccharid wie beispielsweise Stärke, oder um ein Polysaccharidderivat, beispielsweise um carboxyalkylierte Polysaccharide, wie in WO-A-2008/037082 beschrieben.

Die Zusammensetzung des Saug-/Spülkörpers kann zweckmäßigerweise umfassen: ein superabsorbierendes Material der oben genannten Art in einer Menge von 120 bis 170 g/m$^2$, cellulosische Fasern in einer Menge von 120 bis 170 g/m$^2$ und insbesondere thermoplastische Fasern in einer Menge von 5 bis 18 g/m$^2$. Zusätzlich kann auf einer oder beiden Seiten eine Tissueschicht vorgesehen sein. Der Saug-/Spülkörper kann aus den vorgenannten Komponenten (und zusätzlich der salzhaltigen wässrigen Lösung) bestehen.

**EP 3 040 086 B1**

Im Hinblick auf die Ausbildung des faservliesbasierten Saug-/Spülkörpers erweist es sich als vorteilhaft, wenn cellulosische Fasern, vorzugsweise luftgelegte cellulosische Fasern oder vorzugsweise luftgelegte Mischungen aus cellulosischen Fasern und thermoplastischen Fasern, insbesondere Polyolefin-Fasern, insbesondere Polypropylen- oder Polypropylen/Polethylen-Fasern, verwendet werden, die vorzugsweise die gesamte Faserbasis des Saug-/Spülkörpers bilden. Der Anteil der thermoplastischen Fasern einer solchen Fasermischung beträgt vorzugsweise nur etwa 5 bis 10 % des Anteils an cellulosischen Fasern.

Weiterhin erweist es sich als wesentlich, dass hinreichend Flüssigkeit in dem Saug-/Spülkörper über die gesamte Dauer der Verwendung, also bis zum nächsten Verbandwechsel, zur Verfügung steht, damit die eingangs genannte duale Funktion des Saug-/Spülkörpers auch tatsächlich wirksam wird.

Hierfür muss die Verdunstung der Flüssigkeit während der Anwendung der Wundauflage in akzeptablen Grenzen gehalten werden. Im Hinblick darauf erweist es sich als vorteilhaft, wenn die Umhüllung auf der der ersten Schicht abgewandten Seite einen Vliesstoff mit einer außenseitig aufkaschierten Folienschicht aufweist, welche verdunstungshemmend wirkt. Es hat sich gezeigt, dass hierdurch die Verdunstung im Vergleich zu vorbekannten Wundauflagen mit innerhalb der Umhüllung auf der ersten Schicht abgewandten Seite des Saug-der /Spülkörpers angeordneten oder eingelegten Wäscheschutzfolien erheblich reduziert werden kann.

Bei dem Vliesstoff handelt es sich in bevorzugter Weise um einen Polyolefin-Vliesstoff, insbesondere einen Polypropylen-Vliesstoff.

Die Umhüllung auf der der ersten Schicht zugewandten Seite der Wundauflage, aber grundsätzlich auch auf der der ersten Schicht abgewandten Seite der Wundauflage, kann in vorteilhafter Weise ein textiles Flächenmaterial, wie z.B. ein Gestrick, ein Gewirke oder Gewebe, bevorzugt aus Polyolefin, insbesondere aus Polypropylen, sein. Jedenfalls ist die Verwendung einer Umhüllung in Form eines textilen Flächenmaterials, insbesondere Gestricks, Gewirks oder Gewebes, auf der wundzugewandten Seite im Hinblick auf den Flüssigkeitsaustausch, also im Hinblick auf die duale Funktion des Saug-/Spülkörpers, bevorzugt.

Es erweist sich außerdem als vorteilhaft, wenn die flächenspezifische Verdunstungsrate im Zeitintervall 24 Stunden bis 72 Stunden nach der simulierten Aufbringung der zweiten Schicht < 0,020 g/24 h·cm$^2$, insbesondere < 0,017 g/24 h·cm$^2$, insbesondere < 0,015 g/24 h·cm$^2$, insbesondere < 0,012 g/24 h·cm$^2$ ist. Hierfür wird die nachfolgende Testmethode verwandt. Zur Bestimmung der Verdunstungsrate wird eine zunächst wasserdampfdicht verpackte zweite Schicht aus ihrer Umverpackung entnommen. Sie weist einen faservliesbasierten Saug-/Spülkörper mit superabsorbierendem Material, insbesondere FAVOR® PAC 300 von der Firma Evonik Stockhausen GmbH, und typischerweise cellulosische Fasern und gegebenenfalls noch thermoplastische Fasern auf. Vor der wasserdampfdichten Verpackung wurde die zweite Schicht mit salzhaltiger wässriger Lösung, insbesondere Ringerlösung, aktiviert.

Diese zweite Schicht wird sodann mit ihrer der ersten Schicht zugewandten Seite auf eine wasserdampfundurchlässige Folie gelegt und ein umlaufender Randbereich wird mit einer Folie überfangen und so gegen die Unterlage fixiert. Hierfür wird eine Folie (beispielsweise der Handelsmarke OPSITE Flexifix) über die zweite Schicht ausgebreitet, und es wird mittig eine Öffnung in die Abdeckfolie geschnitten, sodass der größte Teil der Oberfläche der zweiten Schicht zur Umgebung exponiert ist und lediglich der umlaufende Randbereich mit 5 mm Breite durch die Abdeckfolie überdeckt bleibt. Somit steht der größte Teil der der ersten Schicht abgewandten Seite der zweiten Schicht zur Umgebung offen, sodass im Saug-/Spülkörper aufgenommene Flüssigkeit über diese Fläche verdampfen kann. Die betreffende Probe wird so bei 23 °C und 50 % relativer Luftfeuchtigkeit gelagert und zu definierten Zeitpunkten, nämlich t = 0 h, t = 24 h, t = 48 h und t = 72 h gewogen. Durch Gewichtsdifferenzbetrachtung kann dann die Verdunstungsrate in g/24 h oder in g/24 h·cm$^2$ angegeben werden. Bei der letzteren Größe handelt es sich um eine auf die Flächeneinheit der exponierten Fläche bezogene flächenspezifische Verdunstungsrate.

Es erweist sich als vorteilhaft, wenn die flächenspezifische Verdunstung innerhalb der ersten 24 Stunden nach der simulierten Aufbringung der zweiten Schicht < 0,050 g/24 h·cm$^2$ Flüssigkeit, insbesondere < 0,037 g/24 h·cm$^2$ Flüssigkeit, insbesondere < 0,025 g/24 h·cm$^2$ Flüssigkeit ist.

Bei einer beispielhaften bevorzugten Zusammensetzung der zweiten Schicht besteht die Faservliesbasis des Saug-/Spülkörpers 4 aus 127 g/m$^2$ Cellulosefasern (Zellstoff), 8 g/m$^2$ Polypropylen/Polyethylen-Fasern als Bindefasern (insbesondere E-505/FV von Firma Schwartswalder). Dieser Fasermischung sind 127 g/m$^2$ der oben genannten superabsorbierenden Polymermaterilien (SAP) homogen beigemischt. Die so erhaltene Mischung (Faservliesbasis + SAP), welche den Saug/Spülkörper bildet, kann zudem von einer cellulosischen Tissueschicht, insbesondere mit einem Flächengewicht von beispielhaft 18 g/m$^2$, auf jeder Seite umgeben sein (Diaper Tissue 1800 von Swedish Tissue AB). Die zweite Schicht kann beispielhaft rund ausgebildet sein, mit einer beispielhaften Abmessung des Saug/Spülkörpers, die im Wesentlichen der Abmessung der zweiten Schicht entspricht. Die Schicht wird schließlich mit 13,6 ml Ringerlösung aktiviert, was vorliegend im Wesentlichen einer Sättigung des Saug-/Spülkörpers mit Flüssigkeit entspricht. Bei einer derartigen Wundauflage wurde eine Verdunstungsrate während der ersten 24 Stunden nach der simulierten Aufbringung von 0,19 g/24 h ermittelt. Hier wäre eine vorteilhafte Obergrenze von 0,8 g/24 h, insbesondere 0,6 g/24 h, insbesondere 0,4 g/24 h zu beachten.

Während des nachfolgenden Zeitintervalls von 24 h bis 72 h betrug die Verdunstung 0,16 g/24 h. Hier wäre eine vorteilhafte Obergrenze von 0,3 g/24 h, insbesondere 0,2 g/24 h, zu beachten.

In einer bevorzugten Ausführungsform kann das Wundsystem in der Epithelisierungsphase der Wundheilung eingesetzt werde. Es hat sich heraus gestellt, dass das Feuchthalten der Wunde in Kombination mit der Anwendung eines geringen elektrischen Stroms eine vorteilhafte Auswirkung auf den Heilungsprozess hat. So kann dieser bevorzugt verkürzt werden und/oder die Narbenbildung kann vorteilhaft reduziert werden.

Ein weiterer Gegenstand der Erfindung ist ein erster und zweiter Stoff zur Verwendung in der therapeutischen Behandlung von Wunden am menschlichen und tierischen Körper, wobei der erste und der zweite Stoff räumlich voneinander getrennt sind, und die ein unterschiedliches Standardpotential $E°$, gemessen bei 25°; 101,3 kPa, pH=0; Ionenaktivität =1, aufweisen, wobei der erste und der zweite Stoff auf einer ersten Schicht aufgebracht ist, die zusammen mit einer zweiten Schicht auf die Wunde aufgebracht wird, und wobei die zweite Schicht die Wunde und den ersten und zweiten Stoff befeuchtet, und wobei die erste Schicht im direkten Kontakt zur Wunde ist. Der erfindungsgemäße erste und der erfindungsgemäße zweite Stoff können somit ein Stoffgemisch darstellen, welches durch chemische und physikalische Interaktion mit menschlichen Körperzellen die Wundheilung positiv beeinflusst.

Erster und zweiter Stoff sowie erste und zweite Schicht weisen bevorzugt die oben beschriebenen Eigenschaften auf. Das heißt, die Erläuterungen zu bevorzugten Ausführungsformen für das erfindungsgemäße System finden auch für die erfindungsgemäßen Stoffe bzw. Schichten Anwendung.

In einer bevorzugten Ausführungsform werden der erste und zweite Stoff zur erfindungsgemäßen Verwendung zur Behandlung von Wunden in der Epithelisierungsphase eingesetzt. Hierbei ergeben sich unerwartet die vorstehend genannten vorteilhaften Effekte bei der Wundheilung.

Wie einleitend beschrieben kann der Heilungsprozess unabhängig von der Art der Wunde in unterschiedliche Phasen typisiert werden. Im Fachgebiet wird zwischen Reinigungs- (Entzündungsphase, Inflammation), Granulations- (Proliferation) und Epithelisierungsphase unterschieden. Gemeinsames Merkmal ist, dass unterschiedliche Zelltypen miteinander interagieren, aktiviert werden und proliferieren.

In der Reinigungsphase wird die Blutung üblicherweise durch die Gerinnungskaskade gestoppt. Es bilden sich üblicherweise Gerinnsel aus Fibrinmolekülen, Fibronektin, Vitronectin und Thrombospondin. Sie können als Leitstruktur für einwandernde Zellen dienen. Gleichzeitig können die Thrombozyten im Gerinnsel Wachstumsfaktoren und Zytokine ausschütten und locken damit immunkompetente Zellen an den Ort der Gewebeverletzung. Es kommt gegebenenfalls zu einer lokalen Entzündung bei der neutrophile Granulozyten eingedrungene Erreger abwehren. Außerdem wird gegebenenfalls abgestorbenes Zellmaterial durch Proteasen beseitigt. In dieser Phase werden meist große Exsudatmengen abgegeben, die die Selbstreinigung der Wunde unterstützen.

Die Granulationsphase ist dadurch gekennzeichnet, dass Monozyten, Endothelzellen und/oder Fibroblasten entlang der provisorischen Fibrinmatrix in das Wundareal einwandern. An den Rändern des frühen Granulationsgewebes können Zellen nekrotisches Gewebe abbauen und am Übergang zum Normalgewebe mit der Neusynthese einer extrazellulären Matrix beginnen. Nach einigen Tagen nimmt die Zellzahl in dieser Matrix massiv zu. Bindegewebe und Blutgefäße werden normalerweise sichtbar. Gegebenenfalls wird neues Kollagen synthetisiert. Während der zweiten Phase werden - sofern erforderlich - neue Gefäße gebildet.

Die Epithelisierungsphase ist, wie vorstehend erwähnt, die dritte Phase der Wundheilung. Sie ist bevorzugt dadurch charakterisiert, dass Epithelzellen in der Wunde erkennbar sind. Bevorzugt überziehen Epithelzellen das noch unfertige Bindegewebe.

Bevorzugt bildet sich, ausgehend vom intakten Epithelgewebe an den Wundrändern, die neue Epidermis. Bevorzugt sorgen ferner Myofibroblasten für eine Kontraktion der Wundränder. Keratinozyten - z.B. vom Wundrand und/oder den Haarwurzelscheiden - können ein neues Epithel bilden.

In einer Ausführungsform ist die Granulationsphase dadurch charakterisiert, dass das Granulationsgewebe wasser- und gefäßärmer wird. Die Kollagenfasern können ausreifen und sich zu Narbengewebe umbilden (Maturation). Es kann dabei gleichzeitig eine Wundkontraktion erfolgen. Bevorzugt haben Fibroblasten ihre Aufbauarbeit beendet und wandeln sich in Fibrozyten und Myofibroblasten um. Die Epithelisierung geht besonders bevorzugt vom Wundrand aus. Hierbei überhäuten bevorzugt die sich neu bildenden Keratinozyten das Granulationsgewebe mit einem zunächst noch sehr feinen Epithel.

Bevorzugt erfolgt die Epithelisierung in einer glatten, feuchten und gut durchbluteten Kriechfläche. Unter Kriechfläche versteht man in diesem Zusammenhang ein ausgebildetes und sauberes Granulationsgewebe, das auf Wundrandniveau reicht. Es sollte im Wesentlichen frei von Nekrosen, Senken, Wundhöhlen oder Hypergranulationen sein. Für die Behandlung einer derartigen Kriechfläche ist das erfindungsgemäße Wundsystem besonders vorteilhaft.

In einer bevorzugten Ausführungsform dauert die Epithelisierungsphase 3 Tage bis 3 Wochen, bevorzugt 5 Tage bis 3 Wochen.

In einer bevorzugten Ausführungsform wird bei der erfindungsgemäßen Verwendung des ersten und zweiten Stoffs die Wunde für 3 bis 5 Tage befeuchtet. Anschließend kann ein Wechsel des Wundsystems erfolgen.

Beschrieben wird die Verwendung eines Kits als Wundauflage, wobei der Kit zwei getrennt voneinander verpackte Schichten umfasst, wobei

(a) die erste trockene Schicht einen ersten und einen zweiten Stoff enthält, wobei der erste und der zweite Stoff räumlich voneinander getrennt sind, sowie ein unterschiedliches Standardpotential $E°$, gemessen bei 25°; 101,3 kPa, pH=0; Ionenaktivität =1, aufweisen; und
(b) die zweite Schicht einen Wassergehalt von 10 bis 80 Gew.% enthält,

wobei bei Anwendung die erste und die zweite Schicht kombiniert werden, so dass die zweite Schicht den ersten und zweiten Stoff der ersten Schicht befeuchtet, wobei die erste Schicht eine Wundkontaktschicht ist, welche im direkten Kontakt zur Wunde ist.

**Patentansprüche**

1.  Wundsystem umfassend:

    (a) eine erste Schicht, enthaltend einen ersten und einen zweiten Stoff, wobei der erste und der zweite Stoff räumlich voneinander getrennt sind, sowie ein unterschiedliches Standardpotential $E°$, gemessen bei 25°; 101,3 kPa, pH=0, Ionenaktivität = 1, aufweisen; und
    (b) eine zweite, die Wunde befeuchtende Schicht,

    wobei die erste Schicht eine Wundkontaktschicht ist, welche im direkten Kontakt zur Wunde ist.

2.  Wundsystem nach Anspruch 1, wobei der erste Stoff und der zweite Stoff jeweils ein Metall und/oder ein entsprechendes Metallsalz sind.

3.  Wundsystem nach Anspruch 1 oder 2, wobei der erste Stoff Zink ist.

4.  Wundsystem nach einem der vorstehenden Ansprüchen 1 bis 3, wobei der zweite Stoff Silber und/oder ein Silbersalz ist.

5.  Wundsystem nach einem der vorstehenden Ansprüchen 1 bis 4, wobei die zweite, die Wunde befeuchtende Schicht (b) eine Abstandsschicht (b1) und eine Spenderschicht (b2), enthaltend einen hydrophilen Polyurethanschaum mit mindestens 10 Gew.-% und höchstens 80 Gew.-% Wasser, umfasst.

6.  Wundsystem Anspruch 5, wobei die Abstandsschicht (b1) eine Hydrogelmatrix, einen Polymerfilm, eine Hydrokolloidmatrix, ein Polymernetz, einen Nonwoven oder ein Adhäsiv umfasst.

7.  Wundsystem nach einem der Ansprüche 5 oder 6, wobei die Spenderschicht (b2) einen hydrophilen Polyurethanschaum mit einem Quellvermögen $\Delta V_1$ von höchstens 80% aufweist.

8.  Wundsystem nach einem der vorstehenden Ansprüche 1 bis 4, wobei die zweite, die Wunde befeuchtende Schicht (b) einen faservliesbasierten Saug/Spülkörper (c), in dem superabsorbierendes Material verteilt aufgenommen ist, und der mit einer salzhaltigen Lösung beaufschlagt ist, umfasst.

9.  Wundsystem nach einem der Ansprüche 1 bis 4 oder 8, wobei der faservliesbasierte Saug-/Spülkörper cellulosische Fasern, insbesondere eine Mischung aus cellulosischen Fasern und thermoplastischen Fasern, insbesondere Polyolefin-Fasern, insbesondere Polypropylen-Fasern oder Polypropylen/Polyethylen-Fasern, umfasst.

10. Erster und zweiter Stoff zur Verwendung in der therapeutischen Behandlung von Wunden am menschlichen oder tierischen Körper, wobei der erste und der zweite Stoff räumlich voneinander getrennt, sowie ein unterschiedliches Standardpotential $E°$, gemessen bei 25°; 101,3 kPa pH=0, Ionenaktivität =1, aufweisen,

    wobei der erste und der zweite Stoff auf einer ersten Schicht aufgebracht sind, die zusammen mit einer zweiten Schicht auf die Wunde aufgebracht wird,
    wobei die zweite Schicht die Wunde und den ersten und zweiten Stoff befeuchtet, und wobei die erste Schicht im direkten Kontakt zur Wunde ist.

11. Erster und zweiter Stoff zur Verwendung nach Anspruch 10, wobei der erste und zweite Stoff zur Behandlung von Wunden in der Epithelisierungsphase eingesetzt wird.

**12.** Erster und zweiter Stoff zur Verwendung nach Anspruch 10 oder 11, wobei die Wunde für 3 bis 5 Tage befeuchtet wird.

**Claims**

**1.** Wound system comprising:

(a) a first layer containing a first and a second substance, said first and second substances being spatially separated from each other and having a different standard potential $E°$ measured at 25°; 101.3 kPa, pH=0, ionic activity = 1; and
(b) a second layer moistening the wound,

wherein the first layer is a wound contact layer which is in direct contact with the wound.

**2.** Wound system according to claim 1, wherein the first substance and the second substance are each a metal and/or a corresponding metal salt.

**3.** Wound system according to claim 1 or 2, wherein the first substance is zinc.

**4.** Wound system according to any one of the preceding claims 1 to 3, wherein the second substance is silver and/or a silver salt.

**5.** Wound system according to any one of the preceding claims 1 to 4, wherein the second wound moistening layer (b) comprises a spacer layer (b1) and a dispenser layer (b2) containing a hydrophilic polyurethane foam with at least 10 wt.% and at most 80 wt.% of water.

**6.** Wound system according to claim 5, wherein the spacer layer (b1) comprises a hydrogel matrix, a polymer film, a hydrocolloid matrix, a polymer net, a non-woven or an adhesive.

**7.** Wound system according to any one of claims 5 or 6, wherein the dispenser layer (b2) comprises a hydrophilic polyurethane foam with a swelling capacity $\Delta V_1$ of at most 80 %.

**8.** Wound system according to any one of the preceding claims 1 to 4, wherein the second wound moistening layer (b) comprises a non-woven fibre-based absorbent/rinsing body (c) in which superabsorbent material is accommodated on a distributed basis and which is acted upon by a salt-containing solution.

**9.** Wound system according to any one of claims 1 to 4 or 8, wherein the fibre fleece-based absorbent/rinsing body comprises cellulose fibres, in particular a mixture of cellulose fibres and thermoplastic fibres, in particular polyolefin fibres, in particular polypropylene fibres or polypropylene/polyethylene fibres.

**10.** First and second substance for use in the therapeutic treatment of wounds on the human or animal body, the first and second substances being spatially separated from each other and having a different standard potential $E°$ measured at 25°; 101.3 kPa, pH=0, ionic activity =1,

wherein the first and second substances are applied to a first layer which is applied to the wound together with a second layer,
wherein the second layer moistens the wound and the first and second substances, and
wherein the first layer is in direct contact with the wound.

**11.** First and second substances for use according to claim 10, wherein the first and second substances are used for treating wounds in the epithelialisation phase.

**12.** First and second substances for use according to claim 10 or 11, wherein the wound is moistened for three to five days.

**Revendications**

**1.** Système de soin pour plaie comportant :

(a) une première couche, contenant une première matière et une seconde matière, dans lequel la première matière et la seconde matière sont spatialement séparées l'une de l'autre, et présentant un potentiel standard $E°$ différent, mesuré à 25 ° ; 101,3 kPa, pH = 0, activité ionique = 1 ; et
(b) une seconde couche humidifiant la plaie,

dans lequel la première couche est une couche de contact avec la plaie qui est en contact direct avec la plaie.

2.  Système de soin pour plaie selon la revendication 1, dans lequel la première matière et la seconde matière sont respectivement un métal et/ou un sel de métal correspondant.

3.  Système de soin pour plaie selon la revendication 1 ou 2, dans lequel la première matière est le zinc.

4.  Système de soin pour plaie selon l'une des revendications 1 à 3 précédentes, dans lequel la seconde matière est l'argent et/ou un sel d'argent.

5.  Système de soin pour plaie selon l'une des revendications 1 à 4 précédentes, dans lequel la seconde couche humidifiant la plaie inclut (b) une couche d'espacement (b1) et une couche donneuse (b2), contenant une mousse de polyuréthane hydrophile ayant au moins 10 % en poids et au plus 80 % en poids d'eau.

6.  Système de soin pour plaie selon la revendication 5, dans lequel la couche d'espacement (b1) comprend une matrice d'hydrogel, un film polymère, une matrice hydrocolloïde, un réseau polymère, un non-tissé ou un adhésif.

7.  Système de soin pour plaie selon l'une des revendications 5 ou 6, dans lequel la couche donneuse (b2) comporte une mousse de polyuréthane hydrophile avec un pouvoir gonflant $\Delta V_1$ d'au plus 80 %.

8.  Système de soin pour plaie selon l'une des revendications 1 à 4 précédentes, dans lequel la seconde couche humidifiant la plaie (b) comprend un corps absorbant/nettoyant à base de nappe de fibres (c), dans lequel une matière super absorbante est reçue de manière répartie et est appliquée avec une solution saline.

9.  Système de soin pour plaie selon l'une des revendications 1 à 4 ou 8, dans lequel le corps absorbant/nettoyant à base de nappe de fibres comprend des fibres cellulosiques, en particulier un mélange de fibres cellulosiques et de fibres thermoplastiques, en particulier des fibres de polyoléfine, en particulier des fibres de polypropylène ou des fibres de polypropylène/polyéthylène.

10. Première et seconde matières pour une utilisation dans le traitement thérapeutique de plaies sur des corps d'humains ou d'animaux, dans lesquelles les première et seconde matières sont spatialement séparées l'une de l'autre, et présentent un potentiel standard $E°$ différent, mesuré à 25 ° ; 101,3 kPa, pH = 0, activité ionique = 1,

dans lesquelles les première et seconde matières sont appliquées sur une première couche qui est appliquée sur la plaie en association avec une seconde couche,
dans lesquelles la seconde couche humidifie la plaie et les première et seconde matières, et
dans lesquelles la première couche est en contact direct avec la plaie.

11. Première et seconde matières pour une utilisation selon la revendication 10, dans lesquelles les première et seconde matières sont utilisées pour le traitement de plaies pendant la phase d'épithélialisation.

12. Première et seconde matières pour une utilisation selon la revendication 10 ou 11, dans lesquelles la plaie est humidifiée pendant 3 à 5 jours.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 201000451 A
- WO 2011141454 A
- WO 2014178945 A
- WO 2008037082 A